# EUROPEAN PATENT APPLICATION

(11) **EP 4 265 728 A1**
(43) Date of publication of application: **25.10.2023**
(21) Application number: 21910897.4
(22) Date of filing: 22.12.2021
(51) Int. Cl.: C12N 15/55, A01H 6/82, C12N 1/20, C12N 1/21, C12N 5/04, C12N 9/16, C12N 15/31, C12N 15/74, C12N 15/82

(54) **DNA CONSTRUCT, VECTOR, BACTERIUM AND METHOD FOR PRODUCING POLYPEPTIDE**

(30) Priority: 24.12.2020 JP 2020215323
(71) Applicant: Denka Company Limited, Tokyo 103-8338 (JP)
(72) Inventor: OGURA Hiroaki, Tokyo 103-8338 (JP); WAKABAYASHI Yuki, Tokyo 103-8338 (JP)
(74) Representative: Hoffmann Eitle
(86) International application number: PCT/JP2021/047678
(87) International publication number: WO 2022/138757

(57) **Abstract**

There is disclosed a DNA construct for expressing a heterologous protein, comprising a DNA encoding a polypeptide, the polypeptide having a His tag added to at least one of the C-terminus and the N-terminus of the following polypeptide (a) or (b), wherein a linker is not present between the His tag and the C-terminal amino acid when the His tag is added to the C-terminus. (a) a polypeptide consisting of an amino acid sequence set forth in SEQ ID NO: 1, 2, or 11; (b) a polypeptide consisting of an amino acid sequence 90% or more identical to the amino acid sequence set forth in SEQ ID NO: 1, 2, or 11, and having phosphodiesterase activity.

## Description

### Technical Field

The present invention relates to DNA constructs, vectors, bacteria, and methods for producing polypeptides. More specifically, the present invention relates to a DNA construct, a vector, and a bacterium for expressing a heterologous protein, and a method for producing a polypeptide by using such a bacterium.

### Background Art

In the expression of a heterologous protein by a gene recombination technique and the purification thereof, when the possibility that an adverse effect on the expression level and/or the function of the purified protein occurs due to the influence on its structure or the like may be expected by addition of a His tag to the C-terminus or N-terminus of the protein or direct addition of a His tag to the C-terminus or N-terminus, inserting a linker between the protein and the His tag is usually performed (for example, Patent Literature 1, Patent Literature 2, and Non Patent Literature 1).

### Citation List

### Patent Literature

Patent Literature 1 International Publication No. WO 2014/139980
Patent Literature 2 International Publication No. WO 2017/106684

### Non Patent Literature

### Non Patent Literature 1

Hochuli et al., "Genetic Approach to Facilitate Purification of Recombinant Proteins with a Novel Metal Chelate Adsorbent" Bio/Technology, Vol 6, Num 11, pp 1321-1325 (1988)

### Summary of Invention

### Technical Problem

The present inventors have found a novel problem: the yield or the specific activity of a phosphodiesterase (PDE) may be reduced when such a DNA construct is used for expressing the phosphodiesterase.

It is an object of the present invention to provide a DNA construct, a vector, and a bacterium by which a high yield and a high specific activity of a phosphodiesterase can be obtained when the phosphodiesterase is expressed heterologously and purified, and a method for producing a polypeptide by using such a bacterium.

### Solution to Problem

As a result of dedicated research to solve the above described novel problem, the present inventors have found that the yield or the specific activity of a phosphodiesterase is reduced when a linker is inserted between the C-terminal amino acid and a His tag, and that the yield or the specific activity of the phosphodiesterase is reduced because a specific amino acid sequence is cleaved by an enzyme comprised in an expression host or a bacterium for transformation of the expression host. Then, the present inventors have found that when a linker is not present between the C-terminal amino acid of a phosphodiesterase and the His tag, a high yield and a high specific activity of the phosphodiesterase can be obtained, and have completed the present invention.

The present invention provides, for example, the following inventions.
[1] A DNA construct for expressing a heterologous protein, comprising a DNA encoding a polypeptide, the polypeptide having a His tag added to at least one of the C-terminus and the N-terminus of the following polypeptide (a) or (b), wherein a linker is not present between the His tag and the C-terminal amino acid when the His tag is added to the C-terminus.
   (a) a polypeptide consisting of an amino acid sequence set forth in SEQ ID NO: 1, 2, or 11;
   (b) a polypeptide consisting of an amino acid sequence 90% or more identical to the amino acid sequence set forth in SEQ ID NO: 1, 2, or 11, and having phosphodiesterase activity.
[2] The DNA construct according to [1], wherein an expression host comprises an enzyme capable of cleaving at least one site of the polypeptide set forth in SEQ ID NO: 3.
[3] The DNA construct according to [1] or [2], wherein the expression host is a plant.
[4] The DNA construct according to any one of [1] to [3], wherein the expression host is a plant of the genus Nicotiana.
[5] A vector comprising the DNA construct according to any one of [1] to [4].
[6] The vector according to [5], wherein the vector is for bacterial transformation.
[7] A bacterium transformed with the vector according to [5] or [6].
[8] The bacterium according to [7], wherein the bacterium comprises an enzyme capable of cleaving at least one site of the polypeptide set forth in SEQ ID NO: 3.
[9] The bacterium according to [7] or [8], wherein the bacterium is an Agrobacterium.
[10] A method for producing the polypeptide according to [1], comprising a step of infecting an expression host or an expression host cell with the bacterium according to any one of [7] to [9].

### Advantageous Effects of Invention

According to the present invention, it is possible to provide a DNA construct, a vector, and a bacterium by which a high yield and a high specific activity of a phosphodiesterase can be obtained when the phosphodiesterase is expressed as heterologous protein and purified, and a method for producing a polypeptide by using such a bacterium.

### Brief Description of Drawings

[Figure 1] Fig. 1 is a schematic diagram showing the cleavage site in the sequence VGRGGGGS (SEQ ID NO: 12) ranging from VGR at the C-terminus of a phosphodiesterase to a linker, the cleavage site being confirmed in Example 1.
[Figure 2] Fig. 2 is a photograph showing the results of evaluation of phosphodiesterase expression by Western blotting in Example 3.

### Description of Embodiments

In one embodiment, the present invention provides a DNA construct for expressing a heterologous protein, comprising a DNA encoding a polypeptide in which a His tag is added to at least one of the C-terminus and the N-terminus of the following polypeptide (a) or (b), wherein when the His tag is added to the C-terminus, a linker is not present between the His tag and the C-terminal amino acid, and when the His tag is added to the N-terminus, a linker may or may not be present between the His tag and the N-terminal amino acid.

In this context, the above described polypeptides are (a) a polypeptide consisting of an amino acid sequence set forth in SEQ ID NO: 1, 2, or 11 and (b) a polypeptide consisting of an amino acid sequence 90% or more identical to the amino acid sequence set forth in SEQ ID NO: 1, 2, or 11, and having phosphodiesterase activity.

Examples of phosphodiesterases derived from bacteria include phosphodiesterases derived from bacteria such as Clostridium perfringens, Listeria monocytogenes, Clostridium botulinum, Bacillus cereus, Helicobacter pylori, and Staphylococcus aureus.

SEQ ID NO: 1 represents the amino acid sequence of the precursor form of a phosphodiesterase protein from an actinomycete. In the amino acid sequence 90% or more identical to the amino acid sequence set forth in SEQ ID NO: 1, the N-terminal amino acid may be methionine, and the C-terminus may be valine-threonine-valine-glycine/alanine-arginine (VTVXR (X = G or A)) (SEQ ID NO: 13).

SEQ ID NO: 2 represents the amino acid sequence of the mature form of the phosphodiesterase protein. In the amino acid sequence 90% or more identical to the amino acid sequence set forth in SEQ ID NO: 2, the N-terminal amino acid may be methionine, and the C-terminus may be valine-threonine-valine-glycine/alanine-arginine (VTVXR (X = G or A)).

SEQ ID NO: 11 represents the amino acid sequence of a phosphodiesterase protein derived from an actinomycete strain different from the actinomycete strain that is the origin of the phosphodiesterase set forth in SEQ ID NO: 1 or 2. In the amino acid sequence 90% or more identical to the amino acid sequence set forth in SEQ ID NO: 11, the N-terminal amino acid may be methionine, and the C-terminus may be valine-threonine-valine-glycine/alanine-arginine (VTVXR (X = G or A)) (SEQ ID NO: 13).

The DNA construct in the present embodiment may be one comprising a DNA encoding a polypeptide in which a His tag is added to the N-terminus of the polypeptide (a) or (b) described above. Examples of such a polypeptide include a polypeptide consisting of an amino acid sequence set forth in SEQ ID NO: 5 or SEQ ID NO: 8. The polypeptide may be one in which a His tag is further added to the C-terminus, such as the polypeptide consisting of the amino acid sequence set forth in SEQ ID NO: 8 (provided that a linker is not present between the His tag and the C-terminal amino acid), or may be one in which a His tag is not added to the C-terminus, such as the polypeptide consisting of the amino acid sequence set forth in SEQ ID NO: 5.

Furthermore, the DNA construct in the present embodiment may be one comprising a DNA encoding a polypeptide in which a His tag is added to the C-terminus of the polypeptide (a) or (b) described above, and a linker is not present between the His tag and the C-terminal amino acid. Examples of such a polypeptide include a polypeptide consisting of an amino acid sequence set forth in SEQ ID NO: 6 or SEQ ID NO: 8. The polypeptide may be one in which a His tag is further added to the N-terminus, such as a polypeptide consisting of the amino acid sequence set forth in SEQ ID NO: 8, or may be one in which a His tag is not added to the N-terminus, such as a polypeptide consisting of the amino acid sequence set forth in SEQ ID NO: 6.

The DNA construct in the present embodiment may be one comprising a DNA encoding a polypeptide consisting of an amino acid sequence set forth in SEQ ID NO: 5, SEQ ID NO: 6, or SEQ ID NO: 8.

In the present specification, the sequence identity to a specific sequence (e.g., SEQ ID NO: 1, 2, 3, 5, 6, 8, or 12) may be, for example, 70% or more, 75% or more, 80% or more, 85% or more, 90% or more, 91% or more, 92% or more, 93% or more, 94% or more, 95% or more, 96% or more, 97% or more, 98% or more, or 99% or more.

The present invention is based on the discovery that a specific amino acid sequence is cleaved (e.g., between VTVXR (SEQ ID NO: 13) and GGG (X = G or A)) when a linker is inserted between the C-terminal amino acid and the His tag. Specifically, when a linker including GGG at its N-terminus (for example, GGGGS (SEQ ID NO: 9)) is present between the His tag and the C-terminal amino acid, the site between C-terminal amino acids VTVXR (X = G or A) (SEQ ID NO: 13) and GGG of the linker is enzymatically cleaved. Thus, when, in the polypeptide in which a His tag is added to at least one of the C-terminus and the N-terminus of polypeptide (a) or (b), the His tag is added to the C-terminus, it is important that a linker is not present between the His tag and the C-terminal amino acid. That is, it is preferable that a linker be not present between the His tag and the C-terminal amino acid, and the His tag be directly linked to the C-terminal amino acid. In contrast, when a His tag is added to the N-terminus, a linker may or may not be present between the His tag and the N-terminal amino acid.

Examples of the linker that may be included between the His tag and the N-terminal amino acid include, but are not particularly limited to, GGGGS (SEQ ID NO: 9), GS, and SG.

A His tag is a short peptide that can be used for purification of a polypeptide by utilizing its property of specifically binding to ions of metals such as nickel. The number of histidine residues included in the His tag may be, for example, 4 to 10, 5 to 7, or 6. The His tag may also be one including methionine at its N-terminus, for example, MHHHHHH (SEQ ID NO: 10).

The DNA construct in the present embodiment is for heterologous protein expression (recombinant protein expression).

The DNA construct in the present embodiment can be transformed into an expression host for heterologous protein expression. Examples of the expression host that can be used include a prokaryote such as a bacterium, a eukaryote such as a yeast, a filamentous fungus, an insect, an animal, algae, and a plant, and a cell thereof.

Examples of the bacterium may include Escherichia coli, a Brevibacillus bacterium, a Corynebacterium bacterium, and a Rhodococcus bacterium.

Examples of the yeast may include yeasts belonging to the genus Saccharomyces, the genus Pichia, the genus Candida, and the genus Torulopsis.

Examples of the filamentous fungus may include filamentous fungi belonging to the genus Aspergillus, the genus Penicillium, and the genus Trichoderma.

Examples of the insect may include insects belonging to the family Noctuidae and the family Bombycidae of the order Lepidoptera.

Examples of the animal include a mammal, and examples of an animal cell may include cells derived from a human cervical cancer, a human fetal kidney, a hamster ovary, and an African green monkey kidney.

Examples of the algae may include algae belonging to the genus Chlamydomonas and the genus Synechococcus.

Examples of the plant include plants belonging to the family Solanaceae (e.g., tobacco, eggplant, tomato, bell pepper, and chili pepper), the family Rosaceae (e.g., rose and strawberry), the family Brassicaceae (e.g., Arabidopsis thaliana, crucifer, Chinese cabbage, cabbage, Japanese radish, and rapeseed), the family Compositae (e.g., chrysanthemum, crown daisy, and lettuce), the family Chenopodiaceae (e.g., spinach and sugar beet), the family Gramineae (e.g., wheat, rice plant, barley, and corn), and the family Leguminosae (e.g., soybean, adzuki bean, common bean, and fava bean). For example, the plant may be a plant of the family Solanaceae or the family Brassicaceae, may be a plant of the genus Nicotiana or a plant of the genus Arabidopsis, or may be Nicotiana benthamiana, Nicotiana tabacum, or Arabidopsis thaliana.

The expression host may be one that comprises an enzyme capable of cleaving at least one site of the polypeptide shown as VTVXRGGG (X = G or A) (SEQ ID NO: 3). It is preferable that the expression host be the one comprising the above described enzyme because the effect of the present invention is more remarkably exhibited in that case.

The DNA construct in the present embodiment may comprise, for example, a promoter sequence, a ribosome binding sequence, a translation initiation site, a polyadenylation tail, a Kozak sequence, a replication origin, a genetic marker, antibiotic resistance, an epitope, a targeting sequence, a reporter gene, and the like, in addition to the DNA encoding the polypeptide.

The DNA construct in the present embodiment can be used for transformation of the expression host by a known method. Examples of the known method may include an electroporation method, a method by using calcium ions, a spheroplast method, a protoplast method, a lithium acetate method, a calcium phosphate method, a lipofection method, and a competent cell method. Examples of a method for expressing a heterologous protein by using a plant as an expression host include an agroinfiltration method, a plant virus vector method, a magnICON (R) system, and a particle gun method.

A vector comprising the DNA construct in the present embodiment can be used for transforming the expression host with the DNA construct of the present embodiment. Examples of the vector include a plasmid, a virus, a cosmid, a lambda phage, and an artificial chromosome. It is preferable that the vector be an expression vector comprising a promoter sequence and the like.

When the expression host is a eukaryote, it is preferable that the vector have a promoter sequence, a ribosome binding sequence, a translation initiation site, a polyadenylation tail, a Kozak sequence, and the like.

When the expression host is a prokaryote such as a bacterium, it is preferable that the vector be capable of self replication in the prokaryote, and have a promoter sequence, a ribosome binding sequence, a translation initiation site, and the like.

In addition to these sequences, the vector can also comprise, for example, a replication origin, a genetic marker, antibiotic resistance, an epitope, a targeting sequence, a reporter gene, and the like.

The expression host may be directly transformed with the vector comprising the DNA construct of the present embodiment, or any cell may be transformed with the vector comprising the DNA construct of the present embodiment and the cell into which the DNA construct has been introduced may be used to transform the expression host. Examples of the cell into which the DNA construct is introduced include a bacterium, a yeast, and an animal cell. The vector for introducing the DNA construct into a cell may be referred to as a vector for cellular transformation, and when the cell is a bacterium, the vector may be referred to as a vector for bacterial transformation. For example, it is possible to express the above described polypeptide, which is a heterologous protein, in an expression host or an expression host cell, by infecting the expression host or the expression host cell with a bacterium transformed with this vector for bacterial transformation.

When the expression host is a plant, examples of the bacterium into which the DNA construct is introduced include bacteria belonging to Agrobacterium species, the genus Rhizobium, the genus Sinorhizobium, the genus Mesorhizobium, the genus Phyllobacterium, the genus Ochrobactrum, and the genus Bradyrhizobium, and it is preferable that the bacterium be Agrobacterium tumefaciens.

The bacterium to be transformed with the vector for bacterial transformation may be one that comprises an enzyme capable of cleaving at least one site of the polypeptide shown as VTVXRGGG (X = G or A) (SEQ ID NO: 3). It is preferable that the bacterium be the one comprising the above described enzyme because the effect of the present invention is more remarkably exhibited in that case.

The case where a plant is used as an expression host is described in more detail below. A method for expressing a polypeptide that is a heterologous protein by using a plant as an expression host can be performed by a known method. Examples of such a method include an agroinfiltration method, a plant virus vector method, a magnICON (R) system, and a particle gun method.

When the agroinfiltration method is used, a plant body expressing a polypeptide that is a heterologous protein can be obtained by transforming an Agrobacterium with the DNA construct of the present embodiment and infecting the plant body with the Agrobacterium. The DNA construct may comprise a T-DNA into which a DNA encoding the polypeptide has been inserted, or the DNA construct may be inserted into the T-DNA of a vector and the Agrobacterium may be transformed therewith.

When the plant virus vector method is used, a plant body expressing a polypeptide that is a heterologous protein can be obtained by inoculating and infecting the plant body with an RNA as a vector, the RNA being obtained from cDNA of a plant virus genome into which the DNA construct of the present embodiment has been inserted. Examples of such a viral vector include a tobacco mosaic virus (TMV) vector, a plum pox virus (PPV) vector, a potato X virus (PVX) vector, an alfalfa mosaic virus (AIMV) vector, a cucumber mosaic virus (CMV) vector, a cowpea mosaic virus (CPMV) vector, and a zucchini yellow mosaic virus (ZYMV) vector.

When the magnICON (R) system is used, a plant body expressing a polypeptide that is a heterologous protein can be obtained by introducing, into a T-DNA vector, cDNA of a TMV or PVX genome into which the DNA construct of the present embodiment has been inserted, and infecting the plant body with an Agrobacterium transformed with the resulting T-DNA vector.

When the particle gun method is used, a plant body expressing a polypeptide that is a heterologous protein can be obtained by ejecting a metal microparticle coated with the DNA construct of the present embodiment at a high speed to introduce the DNA construct into the cell.

In one embodiment, the present invention also provides a method for producing a polypeptide, comprising a step of infecting an expression host or an expression host cell with a bacterium transformed with the above described vector comprising the DNA construct.

The production method of the present embodiment may further comprise a step of culturing the infected expression host or expression host cell. The culture may be performed by using a culture medium in which culturing the host can be performed efficiently. Either a natural medium or a synthetic medium can be used as such a medium. Furthermore, it is preferable that the culture be performed under a condition under which culturing the host can be performed efficiently.

The production method of the present embodiment may further comprise a step of extracting the expressed polypeptide after infection of the expression host or the expression host cell. The extraction of the polypeptide can be performed by a known method depending on the type of the expression host and the polypeptide, and the like. For example, the extraction can be performed by a method based on physical crushing (homogenizer, bead mill, ultrasound, French press, and the like), an osmotic shock method, a freeze-thawing method, a method involving addition of a surfactant, and an enzymatic digestion method. When the expression host is a plant and the plant was infected with the transformed Agrobacterium, the extraction may be performed, for example, by freezing the plant body 5 to 14 days, 7 to 12 days, or 7 days after the infection thereof with the Agrobacterium, and then physically crushing the plant body. Furthermore, for example, the supernatant or precipitate containing the polypeptide of interest may be recovered by centrifugation after physical crushing.

The production method of the present embodiment may further comprise a step of isolating, purifying, and/or concentrating the extracted polypeptide. The isolation, purification, and/or concentration of the polypeptide can be performed by a known method depending on the type of the expression host and the polypeptide, and the like, and can be performed by, for example, affinity, ion exchange, gel filtration, and hydrophobic interaction chromatography.

The yield (%) of the polypeptide produced by the method according to the present embodiment can be 50 to 100%, 50% or more, 59% or more, 70%, 80%, or 90% or more. The yield can be calculated by dividing the amount of the polypeptide after purification (e.g., the concentration of the sample) by the amount of the polypeptide before purification and multiplying the result by 100. The specific activity (U/mg) of the polypeptide produced by the method according to the present embodiment can be 3000 to 6000 U/mg, 3500 to 5300 U/mg, 4000 to 5300 U/mg, or 5000 to 5500 U/mg. The specific activity can be measured by using, for example, Phosphodiesterase Assay Kit (Colorimetric) (manufactured by Abeam plc., Catalog No. ab138876).

The above described specific aspect or the like can be applied, without limitation, as a specific aspect or the like of the method according to the present embodiment.

### Examples

### Example 1: Confirmation of Cleavage Site by Mass Spectrometer

A DNA encoding PDE-GS-His (expression construct 1 described below, SEQ ID NO: 4) was cloned into a tobacco mosaic virus (TMV) vector (manufactured by Icon Genetics GmbH) and the vector was introduced into an Agrobacterium (Agrobacterium tumefaciens) to transform it; after culturing, a mixed solution of the culture solution was infiltrated into a leaf of Nicotiana benthamiana, and the leaf was harvested after about one week. The harvested leaf (infected leaf) was frozen.

A specimen obtained by a similar method to that described below in the section "3. Purification" in Example 3 was used as a sample, and a cleavage site was confirmed by a mass spectrometer (LC-MS) according to the following procedure.

First, the sample was subjected to solvent replacement with ammonium bicarbonate solution. Then, after denaturation with a surfactant, reduction and alkylation were performed. After addition of formic acid, the mixture was subjected to LC-MS measurement. The assignment of the measurement results was performed by using MASCOT Server (Matrix Science).

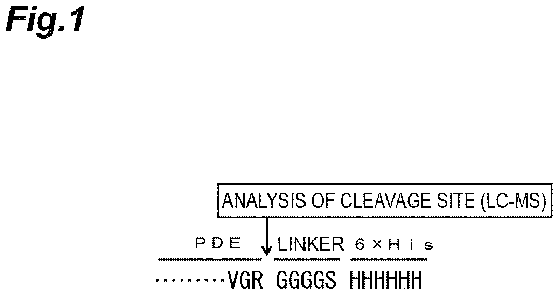

As a result, it was found that, in the VGR at the C-terminus of PDE and the sequence VGRGGGGS (SEQ ID NO: 12) of the linker, the site between R and G adjacent thereto was cleaved (Fig. 1). Further, it was suggested that this cleavage was caused by an enzyme comprised in the Agrobacterium used for protein expression or Nicotiana benthamiana used as an expression host.

### Example 2 Search for Protease Cleaving Specific Site

For PDE-GS-His, Peptide Cutter (https://web.expasy.org/peptide_cutter/) was used to search for a protease candidate cleaving it at the cleavage site of -VTVXR (X = G or A) (cleavage site) GGG-, and as a result, Arg-C proteinase, Clostripain, Thrombin, and Trypsin were extracted.

Arg-C proteinase, Thrombin, and Trypsin belong to the S1 family of proteases, and Clostripain belongs to the C11 family thereof.

Information on proteases of a tobacco plant (N. benthamiana) was obtained from MEROPS (https://www.ebi.ac.uk/merops/index.shtml), and as a result, 18 proteases were extracted as a protease of the S1 family (Table 1), suggesting the possibility that PDE-GS-His has been cleaved by any one of these enzymes.

**[Table 1]**

| Clan (Clan) | Family | Peptidase or Homolog (Subtype) |
|---|---|---|
| PA | S1 | Deg5 chloroplast peptidase |
| PA | S1 | Deg8 chloroplast peptidase |
| PA | S1 | DEG15 peptidase |
| PA | S1 | DEG15 peptidase |
| PA | S1 | At5g36950 |
| PA | S1 | subfamily S1A non-peptidase homologues |
| PA | S1 | subfamily S1B non-peptidase homologues 4 types in total |
| PA | S1 | subfamily S1C unassigned peptidases 6 types in total |
| PA | S1 | subfamily S1D unassigned peptidases 2 types in total |

Information on proteases of a Chinese hamster (Cricetulus griseus) was obtained from MEROPS (https://www.ebi.ac.uk/merops/index.shtml), and as a result, 222 proteases were extracted as a protease of the S 1 family (details omitted). This suggested the possibility that PDE-GS-His was also cleaved at the cleavage site of -VTVXR (X = G or A) (cleavage site) GGG- (SEQ ID NO: 14) when PDE-GS-His was expressed in a Chinese hamster ovary cell (CHO) commonly used for heterologous protein expression by a mammalian cell.

Information on proteases of an E. coli BL21 (DE3) strain (Escherichia coli BL21 (DE3)) was obtained from MEROPS (https://www.ebi.ac.uk/merops/index.shtml), and as a result, 16 proteases were extracted as a protease of the S 1 family (details omitted). This suggested the possibility that PDE-GS-His was also cleaved at the cleavage site of -VTVXR (X = G or A) (cleavage site) GGG- (SEQ ID NO: 14) when PDE-GS-His was expressed in the E. coli BL21 (DE3) strain commonly used for heterologous protein expression by E. coli.

### Example 3 Examination of Tag Sequence for Improving Yield after Purification of Phosphodiesterase

### 1. Generation of vector and expression of protein

The following expression constructs 1 to 5 for expressing a polypeptide having a tag sequence for phosphodiesterase purification were designed.

### Expression construct 1: PDE-GS-His (comparative example)

AAPSLSVMAFNVEQLPSITELAGTPGSDRRAERREAAAVVIKRE
NADVVVLDEAFNKYAQAMRAMELKEVYPYQTSLVGEYCSGSG
RWTSYEGNCSNSPFVVNGGVTLLSKYPILESHQLVYRNSDSKTS
DYKSNKGAALARLSVNGQSVWVAGTHLQADEEGSSPVGNTRK
VRLEQLKELRAWASDKAGGAPVVVAGDFNVEYYTTDPKNGHA
PGTGVSGEVAKANEALGGVLATPGTTGYTYDMVDNPRARLRD
DKDNGYAKYRNRLDYIGYIKGTGTQPSFSDERIVGYDGLRKGG
DPLAEIPSDHQPIVSRVTVGRGGGGSHHHHHH (SEQ ID NO: 4)

### Expression construct 2: His-SG-PDE

HHHHHHSGAAPSLSVMAFNVEQLPSITELAGTPGSDRRAERREA
AAVVIKRENADVVVLDEAFNKYAQAMRAMELKEVYPYQTSLV
GEYCSGSGRWTSYEGNCSNSPFVVNGGVTLLSKYPILESHQLVY
RNSDSKTSDYKSNKGAALARLSVNGQSVWVAGTHLQADEEGSS
PVGNTRKVRLEQLKELRAWASDKAGGAPVVVAGDFNVEYYTT
DPKNGHAPGTGVSGEVAKANEALGGVLATPGTTGYTYDMVDN
PRARLRDDKDNGYAKYRNRLDYIGYIKGTGTQPSFSDERIVGYD
GLRKGGDPLAEIPSDHQPIVSRVTVGR    (SEQ ID NO: 5)

### Expression construct 3: PDE-His

AAPSLSVMAFNVEQLPSITELAGTPGSDRRAERREAAAVVIKRE
NADVVVLDEAFNKYAQAMRAMELKEVYPYQTSLVGEYCSGSG

RWTSYEGNCSNSPFVVNGGVTLLSKYPILESHQLVYRNSDSKTS
DYKSNKGAALARLSVNGQSVWVAGTHLQADEEGSSPVGNTRK
VRLEQLKELRAWASDKAGGAPVVVAGDFNVEYYTTDPKNGHA
PGTGVSGEVAKANEALGGVLATPGTTGYTYDMVDNPRARLRD
DKDNGYAKYRNRLDYIGYIKGTGTQPSFSDERIVGYDGLRKGG
DPLAEIPSDHQPIVSRVTVGRHHHHHH (SEQ ID NO: 6)

### Expression construct 4: His-SG-PDE-GS-His (comparative example)

HHHHHHSGAAPSLSVMAFNVEQLPSITELAGTPGSDRRAERREA
AAVVIKRENADVVVLDEAFNKYAQAMRAMELKEVYPYQTSLV
GEYCSGSGRWTSYEGNCSNSPFVVNGGVTLLSKYPILESHQLVY
RNSDSKTSDYKSNKGAALARLSVNGQSVWVAGTHLQADEEGSS
PVGNTRKVRLEQLKELRAWASDKAGGAPVVVAGDFNVEYYTT
DPKNGHAPGTGVSGEVAKANEALGGVLATPGTTGYTYDMVDN
PRARLRDDKDNGYAKYRNRLDYIGYIKGTGTQPSFSDERIVGYD
GLRKGGDPLAEIPSDHQPIVSRVTVGRGGGGSHHHHHH (SEQ
ID NO: 7)

### Expression construct 5: His-SG-PDE-His

HHHHHHSGAAPSLSVMAFNVEQLPSITELAGTPGSDRRAERREA
AAVVIKRENADVVVLDEAFNKYAQAMRAMELKEVYPYQTSLV
GEYCSGSGRWTSYEGNCSNSPFVVNGGVTLLSKYPILESHQLVY
RNSDSKTSDYKSNKGAALARLSVNGQSVWVAGTHLQADEEGSS
PVGNTRKVRLEQLKELRAWASDKAGGAPVVVAGDFNVEYYTT
DPKNGHAPGTGVSGEVAKANEALGGVLATPGTTGYTYDMVDN
PRARLRDDKDNGYAKYRNRLDYIGYIKGTGTQPSFSDERIVGYD
GLRKGGDPLAEIPSDHQPIVSRVTVGRHHHHHH (SEQ ID NO:

8)

A DNA encoding each expression construct was cloned into a tobacco mosaic virus (TMV) vector (manufactured by Icon Genetics GmbH) and the vector was introduced into an Agrobacterium to transform it; after culturing, a mixed solution of the culture solution was infiltrated into a leaf of Nicotiana benthamiana, and the leaf was harvested after about one week. The harvested leaf (infected leaf) was frozen.

### 2. Expression Study

Three-fold volume of extraction buffer (100 mM Tris, 250 mM NaCl, 5 mM EDTA) was added to the infected leaf, and the leaf was crushed with a bead mill and was allowed to stand on ice for 30 minutes; then, centrifugation (8000 × g, 4°C, 10 minutes) was performed to collect a supernatant and the supernatant was analyzed by SDS-PAGE and Western blotting.

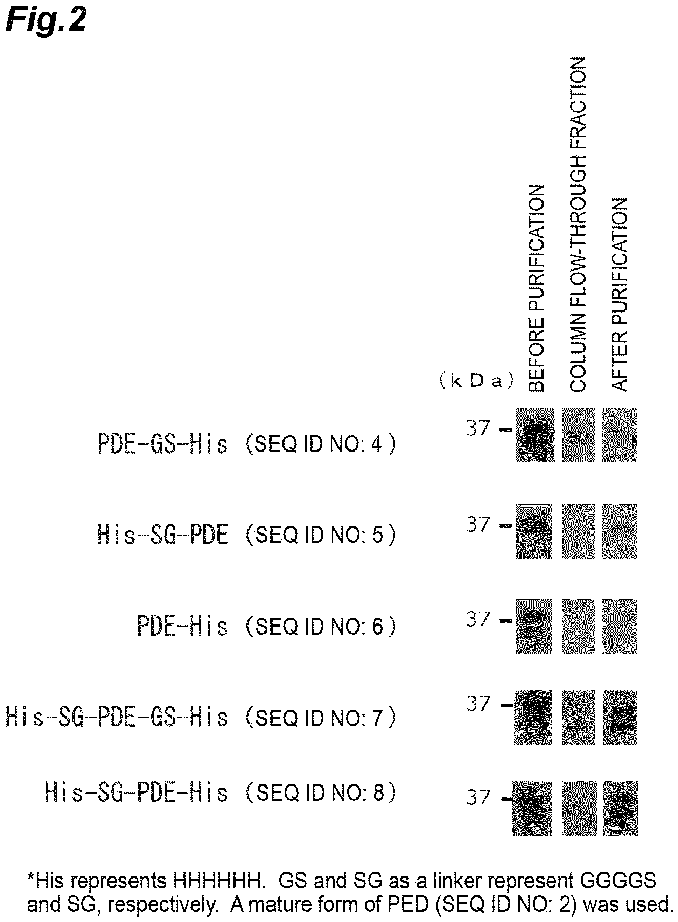

Fig. 2 shows the results of evaluation of expression by Western blotting using an HRP-labeled anti-phosphodiesterase antibody.

### 3. Purification

500 g of the frozen infected leaf was weighed and crushed with a cutting mixer. 250 mL of extraction solution (100 mM Tris, 250 mM NaCl, 40 mM sodium ascorbate) was added to the crushed leaf, and the infected leaf was crushed repeatedly.

The crushed infected leaf collected from the cutting mixer and the crushed infected leaf obtained by washing the inside of the mixer with 250 mL of a fresh extraction solution were combined. This mixture was centrifuged at 12, 000 × g for 5 minutes to collect a supernatant (a fraction from the extraction step).

After adjusting the pH of the fraction from the extraction step, the fraction was filtrated through a 0.45 µm filter, followed by a 0.22 µm filter. The filtrate was subjected to nickel column chromatography (manufactured by GE Healthcare, HisPrep FF16/10 column, Catalog No. 28936551) to remove plant-derived impurities. In this way, a sample solution was obtained.

### 4. Evaluation of yield by ELISA

An anti-PDE antibody was immobilized on a 96-well microplate. After washing the wells, blocking was performed by adding a blocking solution. After a standard solution and the sample solution were added to the wells and allowed to react, an HRP-labeled anti-PDE antibody was added to the wells and allowed to react. An HRP substrate was added to the wells and allowed to react, and a reaction stop solution was added to the wells to stop the reaction. The absorbance of each well at 450 nm was measured, a standard curve was generated based on the results of the standard solution, and the sample concentration was calculated. The yield was calculated from sample concentrations before purification, of the column flow-through fraction, and after purification.

The results of ELISA are shown in Table 2 below (an error of 10%).

**[Table 2]**

| Expression Construct | Before Purification | Column Flow-Through Fraction | After Purification |
|---|---|---|---|
| PDE-GS-His | 100% | 56% | 23% |
| His-SG-PDE | 100% | 9% | 107% |
| PDE-His | 100% | 6% | 70% |
| His-SG-PDE-GS-His | 100% | 11% | 69% |
| His-SG-PDE-His | 100% | 0% | 80% |

| | | | |
|---|---|---|---|
| *His represents HHHHHH. GS and SG as a linker represent GGGGS and SG, respectively. A mature form of PED (SEQ ID NO: 2) was used. | | | |

### 5. Specific Activity Measurement

The specific activity of the purified polypeptide was measured by using Phosphodiesterase assay Kit (manufactured by Abeam plc., Catalog No. ab138876) according to its protocol.

The results of ELISA and the results of specific activity measurement are shown in Table 3 below.

**[Table 3]**

| Expression Construct | Yield (%) | Specific Activity (U/mg) |
|---|---|---|
| PDE-GS-His | 23 | 4952 |
| His-SG-PDE | 107 | 4098 |
| PDE-His | 70 | 5231 |
| His-SG-PDE-GS-His | 69 | 2078 |
| His-SG-PDE-His | 80 | 3680 |

| | | |
|---|---|---|
| *His represents HHHHHH. GS and SG as a linker represent GGGGS and SG, respectively. A mature form of PED (SEQ ID NO: 2) was used. | | |

Thus, it was shown that the yield and specific activity of PDE were improved when the expression construct in which the His tag was not attached to the C-terminus via a linker was used, as compared with when the expression construct in which the His tag was attached to the C-terminus via a linker was used.

## Claims

1. A DNA construct for expressing a heterologous protein, comprising a DNA encoding a polypeptide, the polypeptide having a His tag added to at least one of the C-terminus and the N-terminus of the following polypeptide (a) or (b), wherein a linker is not present between the His tag and the C-terminal amino acid when the His tag is added to the C-terminus;
(a) a polypeptide consisting of an amino acid sequence set forth in SEQ ID NO: 1, 2, or 11;
(b) a polypeptide consisting of an amino acid sequence 90% or more identical to the amino acid sequence set forth in SEQ ID NO: 1, 2, or 11, and having phosphodiesterase activity.

2. The DNA construct according to claim 1, wherein an expression host comprises an enzyme capable of cleaving at least one site of the polypeptide set forth in SEQ ID NO: 3.

3. The DNA construct according to claim 1 or 2, wherein the expression host is a plant.

4. The DNA construct according to any one of claims 1 to 3, wherein the expression host is a plant of the genus Nicotiana.

5. A vector comprising the DNA construct according to any one of claims 1 to 4.

6. The vector according to claim 5, wherein the vector is for bacterial transformation.

7. A bacterium transformed with the vector according to claim 5 or 6.

8. The bacterium according to claim 7, wherein the bacterium comprises an enzyme capable of cleaving at least one site of the polypeptide set forth in SEQ ID NO: 3.

9. The bacterium according to claim 7 or 8, wherein the bacterium is an Agrobacterium.

10. A method for producing the polypeptide according to claim 1, comprising a step of infecting an expression host or an expression host cell with the bacterium according to any one of claims 7 to 9.
